# EUROPEAN PATENT SPECIFICATION

(11) **EP 0 731 786 B1**
(45) Date of publication and mention of the grant of the patent: **21.10.1998**
(21) Application number: 95900765.9
(22) Date of filing: 21.11.1994
(51) Int. Cl.: C07C 235/08, A61K 49/00, C07C 39/367, C07C 59/125, C07C 43/23

(54) **BIPHENYL IODINATED DERIVATIVES AND THEIR DIAGNOSTIC USE**
BIPHENYL JODIERTE DERIVATE UND IHRE VERWENDUNG ALS DIAGNOSTIKUM
DERIVES IODES DE BIPHENYLE ET LEUR UTILISATION DIAGNOSTIQUE

(30) Priority: 03.12.1993 IT MI932542; 23.02.1994 IT MI940324
(43) Date of publication of application: 18.09.1996
(73) Proprietor: BRACCO S.p.A., 20134 Milano (IT); DIBRA S.p.A., I-20122 Milano (IT)
(72) Inventor: UGGERI, Fulvio, I-20079 Codogno (IT); ANELLI, Pier, Lucio, I-20132 Milano (IT); MAFFEZZONI, Constantino, I-16165 Genova (IT)
(74) Representative: Minoja, Fabrizio, Dr.
(86) International application number: EP9403837
(87) International publication number: WO9515307

(56) References cited:
- EP-A- 0 501 875

## Description

This invention refers to new contrast media particularly suitable for the X-ray diagnostic investigation of human and animal body and comprises new compounds of general formula (I): 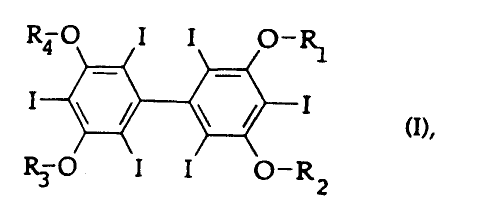 wherein:
- R₁, R₂, R₃, R₄: can be the same or different and are a hydrogen atom or a (C₁-C₆) hydroxyalkyl residue with 1-5 hydroxy groups, or a polyoxaalkyl group of formula 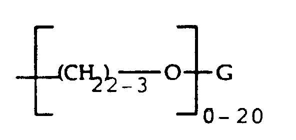
wherein
- G: is H, -CH₃ or -CH₂-CH₃,
or R₁, R₂, R₃, R₄ residues can independently form a group of formula 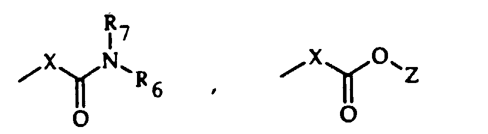 wherein
- X: is equal to a a -(CH₂)₀₋₅-CH-(R₅)- group
wherein
- Z: is a hydrogen atom or a (C₁-C₅) alkyl residue or a (C₁-C₆) hydroxyalkyl residue with 1-5 hydroxy groups,
- R₅: is a hydrogen atom or a CH₃ group,
- R₆ and R₇: which are the same or different, are a hydrogen atom or a (C₁-C₅) alkyl residue or a (C₁-C₆) hydroxyalkyl residue with 1-5 hydroxy groups, or a (C₂-C₆) alkoxyalkyl residue, or are a polyoxaalkyl group as previously defined.

When said compounds of formula (I) contain one or more phenol and/or free acid functions, this invention also comprises the salts thereof with physiologically tolerable organic bases selected from primary, secondary and tertiary amines or basic amino acids or with inorganic bases with cations selected from sodium, potassium, magnesium, calcium or mixtures thereof.

In a particular embodiment the compounds of this invention, which have general formula (II) 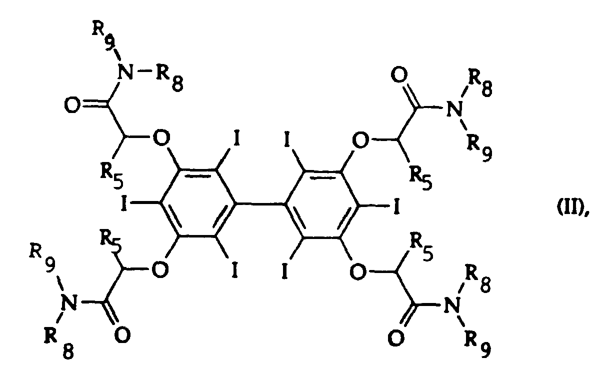 wherein R₅ is defined as in formula (I), R₈ represents a (C₁-C₂) alkyl, or a (C₃-C₄) alkoxyalkyl or a (C₂-C₃) hydroxyalkyl residue and R₉ is hydrogen, are useful intermediates for the preparation of new derivatives of formula (III) 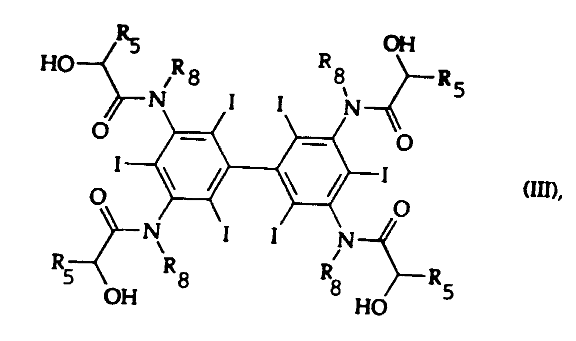 wherein R₅ and R₈ are as previously defined in general formula (II), according to the synthetic process disclosed in patent EP 365541 (Bracco). As a result, these compounds are also included in this invention.

Current X-ray contrast media preferentially contain, as opacifying molecules, neutral polyiodinated aromatic compounds (a particularly updated and exhaustive publication of the state of the art of these diagnostic agents is given by D.P. Swanson et al. "Pharmaceuticals in Medical Imaging", 1990, Mac Millan Publ. Company). The characteristic of these products relies on comprising at least a benzene nucleus in which three hydrogen atoms are substituted by three iodine atoms. A good X-ray absorption requires a high iodine concentration and as a consequence said contrast agents are used in highly concentrated solutions, which can produce undesirable side effects (pain, temperature raising, nausea, pressure lowering and vasal disorders) due to hypertonicity and a higher viscosity compared to blood.

The products of this invention are new iodinated biphenyl derivatives with six iodine atoms on the aromatic nuclei.

This substitution causes, in the case of 3,3',5,5'-tetrahydroxybiphenyl, a iodine concentration of about 78%. Such a high iodine concentration allows the administration of the compounds of this invention in a lower concentration in order to minimize the above mentioned side effects.

Phenol derivatives of this invention have never been disclosed nor suggested up to now. In fact, for instance, patent application EP-A-501875 claims a general formula of this type: 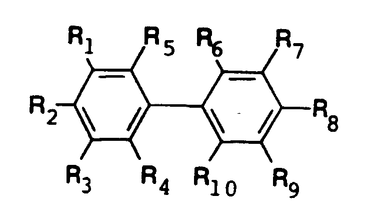 wherein R₁-R₁₀ can be a iodine atom or a group of the following formulae:

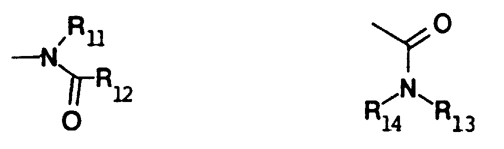

Said derivatives do not absolutely comprise compounds in which there is one or more oxygenated functions directly bound to aromatic rings, nor said application mentions, discloses or claims compounds of formula (III), previously cited.

The products of this invention are characterized generally by high solubility, low viscosity and osmolality, as well as high intravenous, intracisternal and intracerebral tolerability.

The compounds of this invention, in view of their diagnostic use, can be bound to or encapsulated in biomolecules or macromolecules which are aimed at selectively concentrate in the organ or the tissue under examination. Organ selectivity can be reached, for instance, thanks to the encapsulation of said compounds into liposomes.

The compounds of this invention have a wide range of applications, since they can be used for intravasal, (for instance intravenous, intraarterial, intracoronaric, intraventricular administration and so on), intrathecal, intraperitoneal, intralymphatic, intracavital and intraparenchymal administration. Both soluble and less soluble compounds are suitable for oral or enteral administration, and therefore, specifically for the imaging of gastrointestinal (GI) tract. For parenteral administration they can be preferentially formulated as sterile aqueous solutions or suspensions, whose pH can range from 6.0 and 8.5, even thanks to the possible use of physiologically tolerable basic buffering agents (for instance tris[hydroxymethyl]aminomethane or TRIS).

These formulations can also be lyophilized and reconstituted for the use. For the GI use or for injection into body cavities, these agents can be formulated as a solution or suspension containing suitable additives in order, for example, to control viscosity.

For oral administration they can be formulated according to preparation methods routinely used in the pharmaceutical technique: excipients, such as sweeteners or flavouring agents, can be equally added according to known pharmaceutical formulation techniques.

As various changes could be made in the above compositions and methods without departing from the scope of the invention, it is intended that all matter contained in the above description shall be interpreted as illustrative and not in a limiting sense.

### EXAMPLE 1

### A) [1,1'-Biphenyl]-3,3',5,5'-tetrol

To a solution of 1 g of 3,3',5,5'-tetramethoxy-1,1'-biphenyl (prepared according to Kern W. et al., Makromol. Chem., 31, 154-180, 1959) (3.64 mmol) in 10 mL of anhydrous CH₂Cl₂ at room temperature and under nitrogen atmosphere, 1.38 mL of BBr₃ (3.65 g; 14.6 mmol) are dropwise added. Then it is stirred at room temperature for about 5 h. The final solution is slowly poured in 100 mL of a solution of 4% NaHCO₃. The resulting solid is extracted with Et₂O, dried with Na₂SO₄ and evaporated under reduced pressure. 0.8 g of [1,1'-biphenyl]-3,3',5,5'-tetrol (3.64 mmol) are obtained.
- Yield:: 100% m.p. = 284°C (dec.)
- TLC:: silica gel plate 60F 254 Merck
- Eluent:: hexane : CH₂Cl₂ = 100 : 75
- Detector:: UV (254 nm) Rf = 0.25
¹H-NMR, ¹³C-NMR, IR and MS spectra are consistent with the assigned structure.

### B) [2,2',4,4',6,6'-Hexaiodo-1,1'-biphenyl]-3,3',5,5'-tetrol.

To a solution of 1.37 g of compound A) [1,1'-biphenyl]-3,3',5,5'-tetrol (6.027 mmol) in 80 mL of a H₂O/CH₃CN = 1/1 mixture, 15 g of a solution of ICl (45% in I, 16% HCl, 53 mmol I+) are dropwise added in 1 hour at room temperature. After a day at room temperature and two day at 37°C the solution under reduced pressure is concentrated, then diluted with water, and treated with solid Na₂SO₃. The resulting residue is extracted with Et₂O. The crude is purified by flash chromatography. 4.77 g of [2,2',4,4',6,6'-hexaiodo-1,1'-biphenyl]-3,3',5,5'-tetrol (4.92 mmol) are obtained.
- Yield:: 78% m.p. = 200-220 °C (dec.)
¹H-NMR, ¹³C-NMR, IR and MS spectra are consistent with the assigned structure.

### EXAMPLE 2

### 2,2',2",2"'-[[2,2',4,4',6,6'-hexaiodo-1,1'-biphenyl]-3,3',5,5'-tetrayltetrakis(oxy)] tetrakisacetic acid

A suspension of 7.57 g (6 mmol) of methyl tetraester of 2,2',2",2"'-[[2,2',4,4' ,6,6'-hexaiodo-1,1'-biphenyl]-3,3',5,5'-tetrayltetrakis(oxy)]tetrakisacetic acid in 100 mL of H₂O/MeOH = 2/1 (v/v) is adjusted to pH 12 by addition of 2N NaOH. The reaction mixture is stirred at 50°C for 8 h while the pH is kept at 12 by addition of 2N NaOH. After cooling at room temperature MeOH is evaporated and by acidification at pH 1 with 6N HCl a white precipitate is given. After filtration the solid is dissolved in 0.01N NaOH and reprecipitated by addition of 6N HCl. 5.93 g (4.9 mmol) of 2,2',2",2"'-[[2,2',4,4',6,6'-hexaiodo-1,1'-biphe-nyl]-3,3',5,5'-tetrayltetrakis(oxy)]tetrakis acetic acid are obtained as white solid.
- Yield:: 82%
¹H-NMR, ¹³C-NMR, IR and MS spectra are consistent with the assigned structure.

### EXAMPLE 3

### 2,2'-[[3',5'-dihydroxy-2,2',4,4',6,6'-hexaiodo-1,1'-biphenyl]-3,5-diylbis(oxy)]bisacetic acid and 2,2'-[[5,5'-dihydroxy-2,2',4,4',6,6'-hexaiodo-1,1'-biphenyl]-3,3'-diylbis(oxy)] bisacetic acid

A suspension of 19.47 g (20 mmol) of [2,2',4,4',6,6'-hexaiodo-1,1'-biphenyl]-3,3',5,5'-tetrol in 100 mL of DMA, is stirred under nitrogen, is added to 20 mL of a solution 2N of MeONa in MeOH (40 mmol). After 30 min, MeOH is removed by evaporation under reduced pressure and to the reaction mixture, a solution of 6.011 g (40 mmol) of bromomethyl acetate is added in 20 mL of DMA. The reaction mixture is stirred at 80°C for 36 h. The solvent under reduced pressure is evaporated and the residue is diluted in 150 mL of water by adjusting the pH to 12 by addition of 2N NaOH. The reaction mixture is heated at 50° and kept at pH 12 by addition of 2N NaOH until the hydrolysis reaction is completed. After cooling at room temperature, it is acidified at pH 1 with 6N HCl with a resulting precipitation of a solid which is filtered and washed with water. The reaction crude is purified by reverse-preparative HPLC. 4.79 g (4.4 mmol) of 2,2'-[[3',5'-dihydroxy-2,2',4,4',6,6'-hexaiodo-1,1'-biphenyl]-3,5-diylbis(oxy)]bisacetic acid and 3.71 g (3.4 mmol) of 2,2'-[[5,5'-dihydroxy-2,2',4,4',6,6'-hexaiodo-1,1'-biphenyl]-3,3'-diylbis(oxy)]bisacetic acid are obtained. Yield: isomer 3,5: 22% Yield: isomer 3,3: 17% ¹H-NMR, ¹³C-NMR, IR and MS spectra are consistent with the assigned structures.

### EXAMPLE 4

### 2,2',2",2"'-[[2,2',4,4',6,6'-Hexaiodo-1,1'-biphenyl]-3,3',5,5'-tetrayltetrakis(oxy)]tetrakis[N-[2-(2-hydroxyethoxy)ethyl]acetamide]

A solution of 7.57 g (6 mmol) of methyl tetraester of 2,2',2",2"'-[[2,2',4,4',6,6'-hexaiodo-1,1'-biphenyl]-3,3',5,5'-tetrayltetrakis(oxy)]tetrakisacetic acid and 6.030 g (60 mmol) of 2-(2-aminoethoxy)ethanol in 100 mL of MeOH is refluxed for 16 h. After concentration to dryness, the residue is diluted in water and percolated on a cation exchange resin Duolite^{(R)} C 20 MB and an anion exchange resin Duolite^{(R)} A 30 B. The neutral eluate is concentrated to dryness and the residue is purified by silica gel chromatography. 5.18 g (3.4 mmol) of 2,2',2",2"'-[[2,2',4,4',6,6'-hexaiodo-1,1'-biphenyl]-3,3',5,5'-tetrayltetrakis(oxy)]tetrakis[N-[2-(2-hydroxyethoxy)e-thyl]acetamide] are obtained as white solid.
- Yield:: 56%
¹H-NMR, ¹³C-NMR, IR and MS spectra are consistent with the assigned structure.

### EXAMPLE 5

### 2,2',2",2"'-[[2,2',4,4',6,6'-Hexaiodo-1,1'-biphenyl]-3,3',5,5'-tetrayltetrakis(oxy-2,1-ethanediyloxy-2,1-ethanediyloxy)]tetrakisethanol

A suspension of 9.73 g (10 mmol) of [2,2',4,4',6,6'-hexaiodo-1,1'-biphenyl]-3,3',5,5'-te-trol in 100 mL of DMA, stirred under nitrogen, is added to 22 mL of a solution 2N of MeONa in MeOH (44 mmol). After 30 min, MeOH is removed by evaporation under reduced pressure and to the reaction mixture 3.0 g (20 mmol) of NaI and a solution of 7.42 g (44 mmol) of 2-[2-(2-chloroethoxy)ethoxy]ethanol are added. The reaction mixture is heated for 90 h at 80°C. After cooling at room temperature, the precipitate is filtered and the solvent is evaporated under reduced pressure. The oily residue is treated with CH₂Cl₂ to remove the last traces of DMA, then is purified by silica gel chromatography. 6.028 g (4.8 mmol) of 2,2',2",2"'-[[2,2',4,4',6,6'-hexaiodo-1,1'-biphenyl]-3,3',5,5'-tetrayltetrakis(oxy-2,1-ethanediyloxy-2,1-ethanediyloxy)]tetrakisethanol are obtained.
- Yield:: 48%
¹H-NMR, ¹³C-NMR, IR and MS spectra are consistent with the assigned structure.

## Claims

1. Compounds of general formula (I) 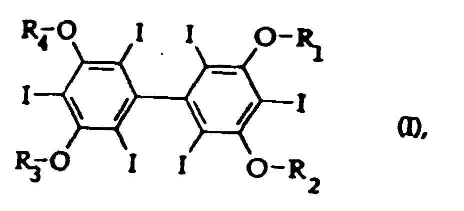 wherein
R₁, R₂, R₃, R₄ can be the same or different and are a hydrogen atom or a (C₁-C₆) hydroxyalkyl residue with 1-5 hydroxy groups, or a polyoxaalkyl group of formula 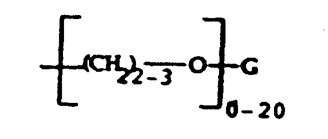
wherein
G is H, -CH₃ or -CH₂-CH₃,
or R₁, R₂, R₃, R₄ residues can independently form a group of formula 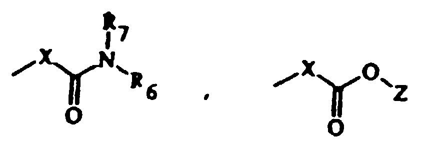 wherein
X is equal to a a -(CH₂)₀₋₅-CH-(R₅)- group
wherein
Z is a hydrogen atom or a (C₁-C₅) alkyl residue or a (C₁-C₆) hydroxyalkyl residue with 1-5 hydroxy groups,
R₅ is a hydrogen atom or a CH₃ group,
R₆ and R₇ which are the same or different, are a hydrogen atom or a (C₁-C₅) alkyl residue or a (C₁-C₆) hydroxyalkyl residue with 1-5 hydroxy groups, or a (C₂-C₆) alkoxyalkyl residue, or are a polyoxaalkyl group as previously defined and,
in case said compounds of formula (I) contain one or more phenol and/or free acid functions, also the salts thereof with physiologically tolerable organic bases selected from primary, secondary and tertiary amines or basic amino acids or with inorganic bases which cations are selected from sodium, potassium, magnesium, calcium or mixtures thereof.

2. Contrast medium particularly useful for X-ray diagnosis of human or animal body comprising at least one compound of general formula (I), according to claim 1 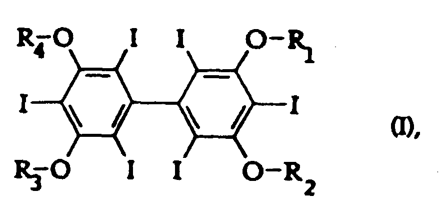 wherein R₁, R₂, R₃, R₄ are as previously defined, as well as the salts thereof with physiologically tolerable organic bases selected from primary, secondary and tertiary amines or basic amino acids or with inorganic bases which cations are selected from sodium, potassium, magnesium, calcium or mixtures thereof.

3. Compounds according to claim 1 of general formula (II) 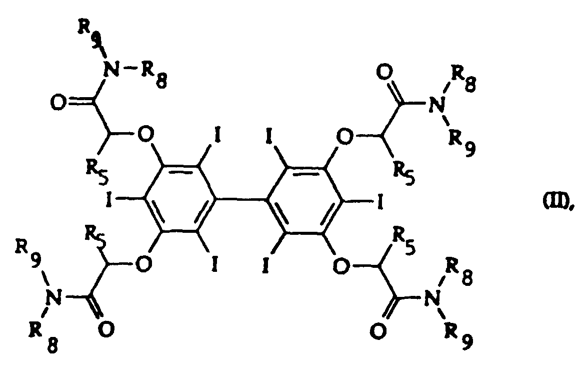 wherein
R₅ is a hydrogen atom or a CH₃ group,
R₈ represents a (C₁-C₂) alkyl, or a (C₃-C₄) alkoxyalkyl, or a (C₂-C₃) hydroxyalkyl residue,
R₉ is a hydrogen atom.

4. Compounds of general formula (III) 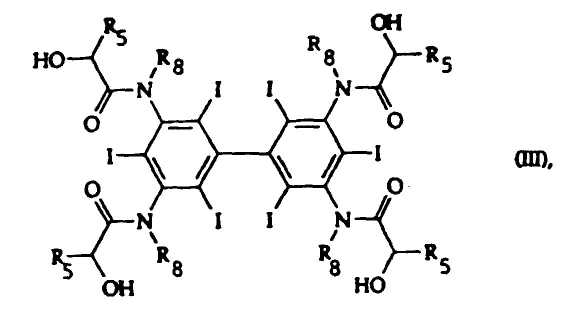 wherein R₅ is hydrogen or CH₃ group and R₈ is a (C₁-C₂) alkyl, or (C₃-C₄) alkoxyalkyl or (C₂-C₃) hydroxyalkyl residue.

5. Contrast medium particularly useful for X-ray diagnosis of human or animal body comprising at least one compound of general formula (III), according to claim 4 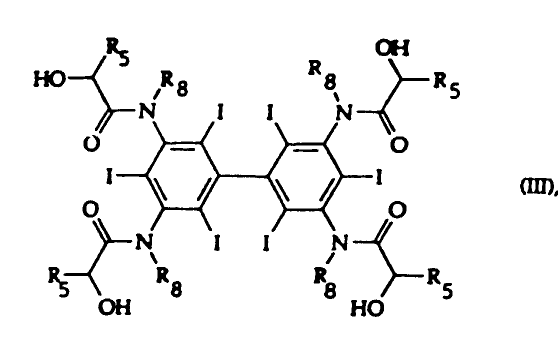 wherein R₅ and R₈ have the same meaning as set forth in claim 4.

6. Use of at least one of the compounds of general formula (I) and/or (III) and the salts thereof for the preparation of X-ray diagnostic formulations.

7. Use of compounds of formula (II), according to claim 3, for the preparation of the corresponding compounds of formula (III).

## Patentansprüche

1. Verbindungen der allgemeinen Formel (I) 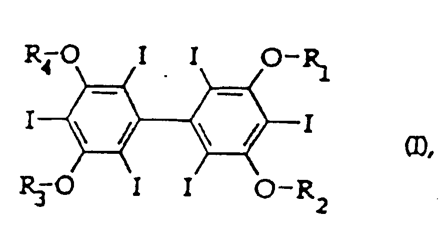 wobei R₁, R₂, R₃, R₄ gleich oder verschieden sein können und ein Wasserstoffatom oder ein (C₁-C₆)Hydroxyalkylrest mit 1 bis 5 Hydroxygruppen oder eine Polyoxaalkylgruppe der Formel 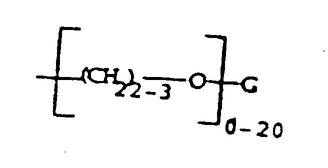 sind, wobei
G H, -CH₃ oder -CH₂-CH₃ ist,
oder die Reste R₁, R₂, R₃, R₄ unabhängig voneinander eine Gruppe der Formel 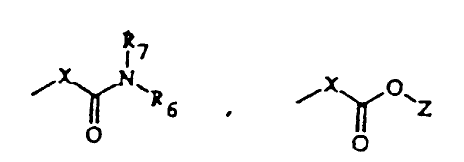 bilden können, wobei
X gleich einer -(CH₂)_{O-5}-CH-(R₅)-Gruppe ist, wobei
Z ein Wasserstoffatom oder ein (C₁-C₅)Alkylrest oder ein (C₁-C₆)Hydroxyalkylrest mit 1 bis 5 Hydroxygruppen ist,
R₅ ein Wasserstoffatom oder eine CH₃-Gruppe ist,
R₆ und R₇, die gleich oder verschieden sind, ein Wasserstoffatom oder ein (C₁-C₅)Alkylrest oder ein (C₁-C₆)Hydroxyalkylrest mit 1 bis 5 Hydroxygruppen oder ein (C₂-C₆)Alkoxyalkylrest oder eine wie zuvor definierte Polyoxaalkylgruppe sind, und in dem Fall von Verbindungen der Formel (I), die eine oder mehr Phenol- und/oder freie Säurefunktionen enthalten, auch die Salze davon mit physiologisch verträglichen organischen Basen, die ausgewählt werden aus primären, sekundären und tertiären Aminen oder basischen Aminosäuren oder mit anorganischen Basen, deren Kationen ausgewählt werden aus Natrium, Kalium, Magnesium, Calcium oder Mischungen davon.

2. Insbesondere für die Röntgen-Diagnose des menschlichen oder tierischen Körpers geeignetes Kontrastmedium, umfassend mindestens eine Verbindung der allgemeinen Formel (I) nach Anspruch 1 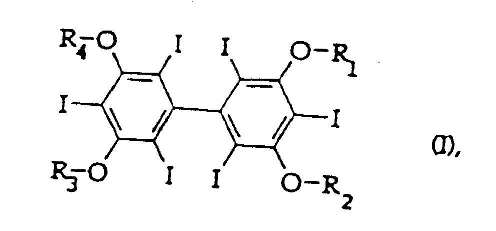 wobei R₁, R₂, R₃, R₄ wie zuvor definiert sind, genauso wie die Salze davon mit physiologisch verträglichen organischen Basen, die ausgewählt werden aus primären, sekundären und tertiären Aminen oder basischen Aminosäuren oder mit anorganischen Basen, deren Kationen ausgewählt werden aus Natrium, Kalium, Magnesium, Calcium oder Mischungen davon.

3. Verbindungen nach Anspruch 1 der allgemeinen Formel (II) 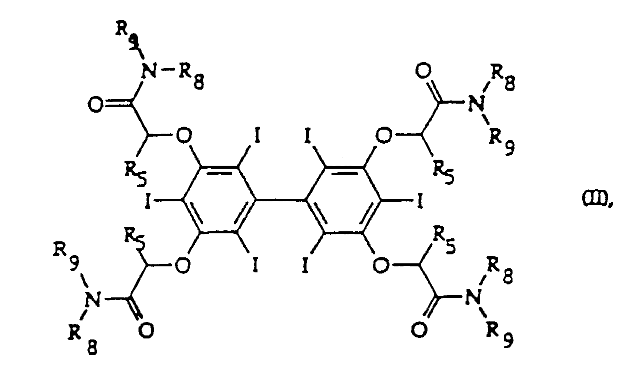 wobei
R₅ ein Wasserstoffatom oder eine CH₃-Gruppe ist,
R₈ einen (C₁-C₂)Alkyl-, oder einen (C₃-C₄) Alkoxyalkyl- oder einen (C₂-C₃)Hydroxyalkylrest darstellt,
R₉ ein Wasserstoffatom ist.

4. Verbindungen der allgemeinen Formel (III) 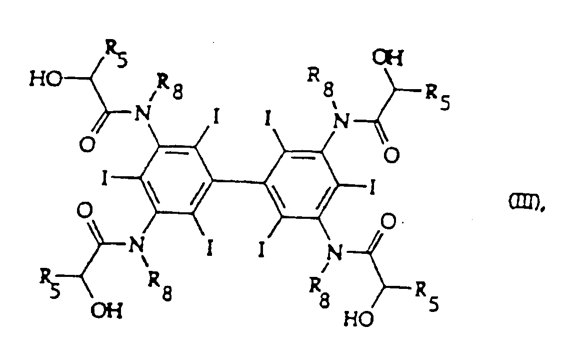 wobei R₅ Wasserstoff oder CH₃-Gruppe ist und R₈ ein (C₁-C₂)Alkyl-, oder (C₃-C₄)Alkoxyalkyl- oder (C₂-C₃)Hydroxyalkylrest ist.

5. Insbesondere für die Röntgen-Diagnose des menschlichen oder tierischen Körpers geeignetes Kontrastmedium, umfassend mindestens eine Verbindung der allgemeinen Formel (III) nach Anspruch 4 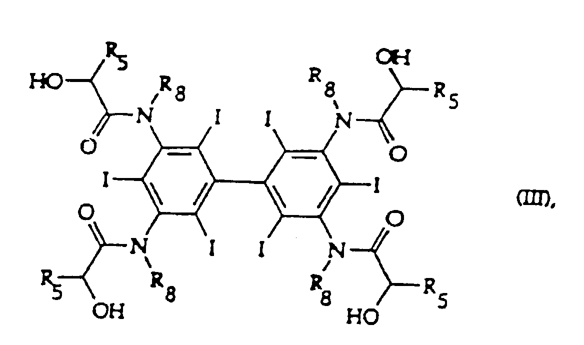 wobei R₅ und R₈ dieselbe Bedeutung haben wie in Anspruch 4 angegeben.

6. Verwendung von mindestens einer der Verbindungen der allgemeinen Formel (I) und/oder (III) und von deren Salze für die Herstellung von röntgendiagnostischen Formulierungen.

7. Verwendung von Verbindungen der Formel (II) entsprechend Anspruch 3 zur Herstellung der entsprechenden Verbindungen der Formel (III).

## Revendications

1. Composés de formule générale (I) 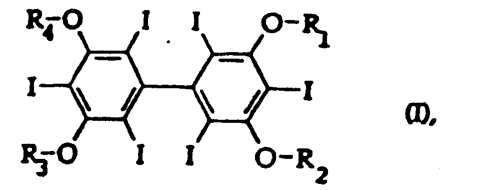 dans laquelle
R₁, R₂, R₃, R₄ peuvent être identiques ou différents et sont un atome d'hydrogène ou un résidu hydroxyalkyle en C₁ à C₆ ayant 1 à 5 groupes hydroxy, ou un groupe polyoxa-alkyle de formule 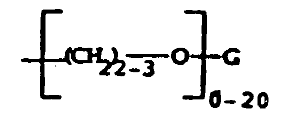 dans laquelle
G est H, -CH₃ ou -CH₂-CH₃,
ou bien les résidus R₁, R₂, R₃, R₄ peuvent indépendamment former un groupe de formule 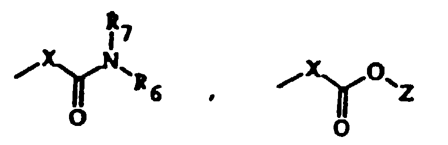 dans laquelle
X est égal à un groupe -(CH₂)₀₋₅-CH-(R₅)- dans lequel Z est un atome d'hydrogène ou un résidu alkyle en C₁ à C₅ ou un résidu hydroxyalkyle en C₁ à C₆ ayant 1 à 5 groupes hydroxy,
R₅ est un atome d'hydrogène ou un groupe CH₃,
R₆ et R₇, qui sont identiques ou différents, sont un atome d'hydrogène ou un résidu alkyle en C₁ à C₅ ou un résidu hydroxyalkyle en C₁ à C₆ ayant 1 à 5 groupes hydroxy, ou un résidu alcoxyalkyle en C₂ à C₆, ou sont un groupe polyoxa-alkyle tel que défini précédemment, et
dans le cas où lesdits composés de formule (I) contiennent une ou plusieurs fonctions phénol et/ou acide libre, également les sels de ceux-ci avec des bases organiques physiologiquement tolérables choisies parmi les amines primaires, secondaires et tertiaires ou les acides aminés basiques, ou avec des bases inorganiques ayant des cations choisis parmi les cations sodium, potassium, magnésium, calcium ou des mélanges de ceux-ci.

2. Milieu de contraste particulièrement utile pour l'étude diagnostique par rayons X d'un corps humain ou animal, comprenant au moins un composé de formule générale (I), selon la revendication 1 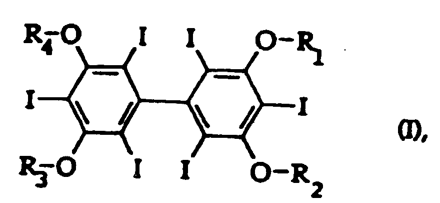 dans laquelle R₁, R₂, R₃, R₄ sont tels que définis précédemment, ainsi que les sels de celui-ci avec des bases organiques physiologiquement tolérables choisies parmi les amines primaires, secondaires et tertiaires ou les acides aminés basiques, ou avec des bases inorganiques ayant des cations choisis parmi les cations sodium, potassium, magnésium, calcium ou des mélanges de ceux-ci.

3. Composés selon la revendication 1 de formule générale (II) 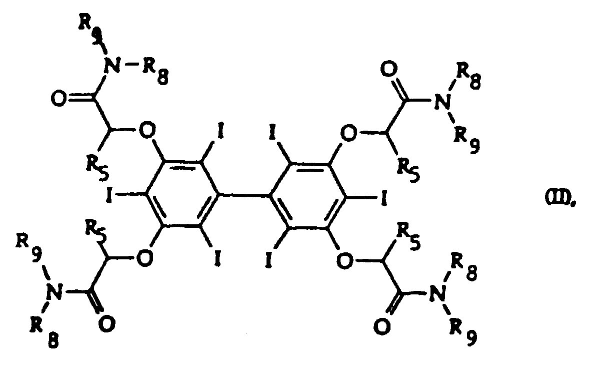 dans laquelle
R₅ est un atome d'hydrogène ou un groupe CH₃,
R₈ représente un groupe alkyle en C₁ à C₂, ou un groupe alcoxyalkyle en C₃ à C₄, ou un résidu hydroxyalkyle en C₂ à C₃,
R₉ est un atome d'hydrogène.

4. Composés de formule générale (III) 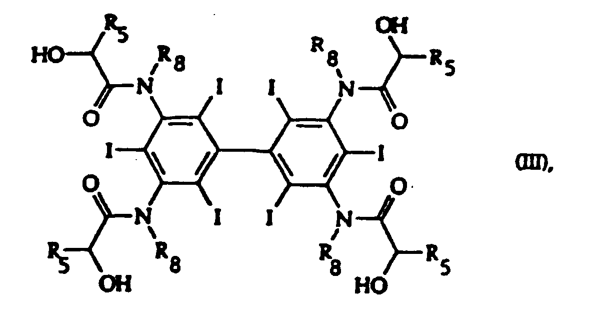 dans laquelle R₅ est un hydrogène ou un groupe CH₃ et R₈ est un groupe alkyle en C₁ à C₂, ou un groupe alcoxyalkyle en C₃ à C₄, ou un résidu hydroxyalkyle en C₂ à C₃.

5. Milieu de contraste particulièrement utile pour l'étude diagnostique par rayons X d'un corps humain ou animal, comprenant au moins un composé de formule générale (III), selon la revendication 4 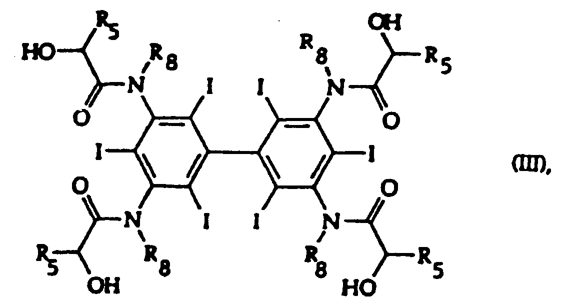 dans laquelle R₅ et R₈ ont la même signification que celle présentée dans la revendication 4.

6. Utilisation d'au moins l'un des composés de formule générale (I) et/ou (III) et des sels de ceux-ci pour la préparation de formulations diagnostiques par rayons X.

7. Utilisation de composés de formule (II), selon la revendication 3, pour la préparation des composés de formule (III) correspondants.
